# EUROPEAN PATENT SPECIFICATION

(11) **EP 4 098 298 B1**
(45) Date of publication and mention of the grant of the patent: **27.08.2025**
(21) Application number: 21776305.1
(22) Date of filing: 03.02.2021
(51) Int. Cl.: A61M 5/31, A61M 5/00

(54) **SYRINGE-BARREL GRIP, BARREL ASSEMBLY, AND SYRINGE**
SPRITZENZYLINDERGRIFF, ZYLINDEREINHEIT UND SPRITZE
POIGNÉE DE CYLINDRE DE SERINGUE, ENSEMBLE CYLINDRE ET SERINGUE

(30) Priority: 24.03.2020 JP 2020052233
(43) Date of publication of application: 07.12.2022
(73) Proprietor: TERUMO Kabushiki Kaisha, Tokyo 151-0072 (JP)
(72) Inventor: MATSUMOTO Fumiya, Fujinomiya-shi, Shizuoka 418-0004 (JP)
(74) Representative: Casalonga
(86) International application number: PCT/JP2021/003898
(87) International publication number: WO 2021/192637

(56) References cited:
- WO-A1-2004/091700
- WO-A1-2017/039786
- JP-A- 2004 097 277
- JP-A- 2014 028 114
- JP-A- 2015 080 688
- JP-A- 2017 527 406
- JP-A- 2019 510 589

## Description

### Technical Field

The present invention relates to a syringe barrel grip attached to a syringe barrel, and a barrel assembly and a syringe each including the syringe barrel grip. WO 2004/091700 A1 already discloses a syringe grip, and when attached to said grip, flanges of a syringe are inserted and then rotated relative to the grip within receiving portions to enter the guide groove and contact an end wall which thereafter restricts rotation.JP2004 097277 A discloses a syringe grip and when a syringe barrel is attached to the grip, the syringe barrel is clipped into the mounting hole and shaft hole such that rotation is restricted as a flange contacts side walls of the grip, WO2017/039786 A1 relates to a syringe grip which is clipped onto a syringe through translation. It would be desirable to provide an alternative way of attaching a grip to a syringe and of restricting rotation of the syringe with regard to the grip.

### Background Art

Some drug-filled syringes (prefilled syringes) each use a plunger that is not coupled to a gasket in order to prevent medical errors. To such a prefilled syringe, a clip (backstop) for preventing a plunger from moving to a proximal end side and falling off from a barrel is attached (Patent Literature 1).

In addition, in order to facilitate gripping and operation of a syringe, it is also known to attach a clip (grip) including a finger hook portion protruding outward to a proximal end portion of a barrel (Patent Literature 2).

These clips are each fitted to an outer peripheral surface of a barrel at a cylindrical fitting portion and firmly attached to a syringe so as not to rattle or come off during operation of the syringe.

### Citation List

### Patent Literature

Patent Literature 1: JP 2015-517860 A (US 2015-105734 A1 and US 2019-365565 A1)
Patent Literature 2: WO 2017/073658 A (US 2018-243509 A1)

### Summary of Invention

### Technical Problem

In recent years, in ophthalmic use and the like, higher sterility of not only a drug to be administered and a portion in contact with the drug but also the entire prefilled syringe including a grip is required. Therefore, the present inventors have studied an application of a surface sterilization method with a small heat load, such as hydrogen peroxide sterilization, EOG sterilization, or NO₂ sterilization, to a prefilled syringe to which a grip is attached. These surface sterilization methods perform sterilization with a gas (sterilization gas), and in order to achieve higher sterility, the sterilization gas preferably comes into contact with a wider area of a surface of the prefilled syringe.

Therefore, the present invention provides a syringe barrel grip having good operability and capable of achieving higher sterility in a state of being attached to a syringe barrel, and a barrel assembly and a syringe each including such a syringe barrel grip.

### Solution to Problem

The above object is achieved by the following.

A syringe barrel grip attached to a syringe barrel including a cylindrical body and a flange disposed in the cylindrical body, protruding outward, and including a pair of flange straight portions facing each other in parallel,
the grip including a flange housing portion that houses the flange, in which
the flange housing portion includes: a side surface opening formed on a side surface of the grip; a flange insertion portion extending in an internal direction of the grip from the side surface opening; and a flange rotation restricting portion that communicates with the flange insertion portion, extends into the grip, and restricts relative rotation between the grip and the flange that has entered the flange rotation restricting portion by rotating the barrel by a predetermined angle in a state where the flange is housed in the flange insertion portion,
the flange insertion portion includes a first pair of grip straight portions having a slightly wider width than the pair of flange straight portions, and the flange rotation restricting portion includes a second pair of grip straight portions having a slightly wider width than the pair of flange straight portions and is inclined with respect to the first pair of grip straight portions,
in a state where the flange is housed in the flange rotation restricting portion, front sides of the pair of flange straight portions are located in the second pair of grip straight portions, and rear sides of the pair of flange straight portions are located in the first pair of grip straight portions, and
in a state where the flange of the barrel is housed in the flange rotation restricting portion of the grip, a lower inner peripheral surface of the grip is not in contact with the barrel, and there is a gap for allowing a sterilization gas to flow between the lower inner peripheral surface of the grip and the barrel.

The above object is also achieved by the following.

A barrel assembly including: a syringe barrel including a cylindrical body and a flange disposed at a proximal end of the cylindrical body, protruding outward, and including a pair of flange straight portions facing each other in parallel; and the syringe barrel grip attached to the barrel.

The above object is also achieved by the following.

A syringe including: the barrel assembly; a gasket slidably housed in the barrel; and a plunger for moving the gasket.

### Brief Description of Drawings

Fig. 1 is a front view illustrating an embodiment of a syringe of the present invention.
Fig. 2 is a cross-sectional view taken along line A-A in Fig. 1.
Fig. 3 is a cross-sectional view taken along line B-B in Fig. 1.
Fig. 4 is an enlarged view of a portion D in Fig. 3.
Fig. 5 is a cross-sectional view taken along line C-C in Fig. 2.
Fig. 6 is an explanatory view for explaining operation of the syringe illustrated in Fig. 1.
Fig. 7 is a front view of a syringe barrel grip used in the syringe of Fig. 1.
Fig. 8 is a plan view of the syringe barrel grip used in the syringe of Fig. 1.
Fig. 9 is a bottom view of the syringe barrel grip used in the syringe of Fig. 1.
Fig. 10 is a cross-sectional view taken along line E-E in Fig. 7.
Fig. 11 is a cross-sectional view taken along line F-F in Fig. 7.
Fig. 12 is a cross-sectional explanatory view corresponding to Fig. 10 for explaining a process for manufacturing the syringe of Fig. 1.
Fig. 13 is a cross-sectional explanatory view corresponding to Fig. 10 for explaining the process for manufacturing the syringe of Fig. 1.
Fig. 14 is a cross-sectional explanatory view corresponding to Fig. 10 for explaining the process for manufacturing the syringe of Fig. 1.
Fig. 15 is a cross-sectional partial explanatory view corresponding to Fig. 5 for explaining a syringe barrel grip according to another embodiment of the present invention.
Fig. 16 is a cross-sectional partial explanatory view corresponding to Fig. 5 for explaining a syringe barrel grip according to another embodiment of the present invention.
Fig. 17 is a cross-sectional explanatory view corresponding to Fig. 10 for explaining a syringe barrel grip according to another embodiment of the present invention.
Fig. 18 is a cross-sectional explanatory view corresponding to Fig. 10 for explaining a syringe barrel grip according to another embodiment of the present invention.

### Description of Embodiments

A syringe barrel grip of the present invention, and a barrel assembly and a syringe each including such a syringe barrel grip will be described with reference to an embodiment illustrated in the drawings.

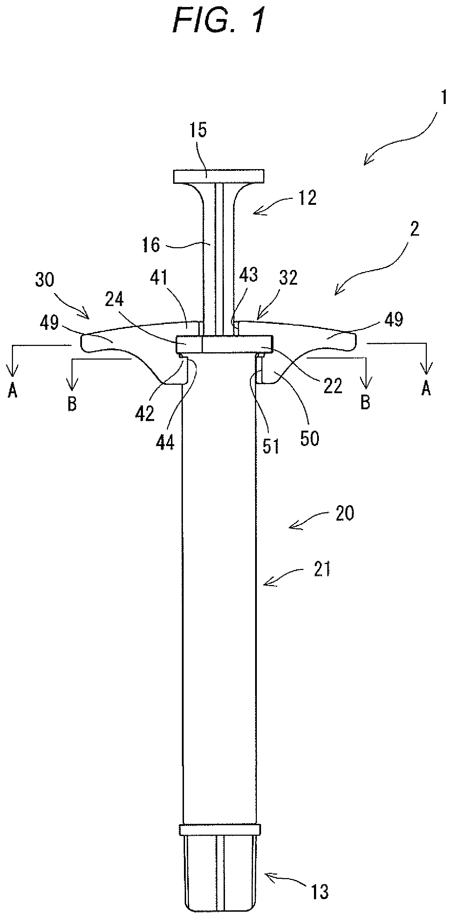

Note that the present embodiment will be described by defining an upper side in Fig. 1 (the side on which a flange 22 of a syringe barrel 20 is formed) as a proximal end side or an upper side, defining a lower side in Fig. 1 (the side on which a nozzle portion 23 of the syringe barrel 20 is disposed) as a distal end side or a lower side, and defining the vertical direction in Fig. 1 as an axial direction (the axial direction of the syringe barrel 20 or a cylindrical body 21).

As illustrated in Figs. 1 to 6, a syringe 1 of the present invention includes: a barrel assembly 2 including a syringe barrel 20 (hereinafter, also simply referred to as a barrel 20) and a syringe barrel grip 30 (hereinafter, also simply referred to as a grip 30) attached to the barrel 20; a gasket 11 slidably housed in the barrel 20; and a plunger 12 for moving the gasket 11.

The barrel 20 includes a cylindrical body 21 and a flange 22 (thickness t) disposed in the cylindrical body 21, protruding outward (in a direction orthogonal to the axial direction of the barrel 20), and including a pair of flange straight portions 24 and 24 (width w) facing each other in parallel. In the present embodiment, the cylindrical body 21 of the barrel 20 has a circular (annular) outer shape and a circular (annular) inner shape in a cross section taken along a plane orthogonal to the axial direction thereof as a whole. A distal end opening portion (nozzle portion) 23 for discharging a drug is disposed at a distal end portion of the barrel 20.

In addition, the barrel 20 includes a collar 25 covering a proximal end side portion of the nozzle portion 23. A barrel side screwing portion (female screwing portion) is formed on an inner surface of the collar 25.

The nozzle portion 23 is sealed by a seal cap 13 to prevent leakage of a drug 60 stored in the barrel 20 and to prevent the aseptically filled drug 60 from coming into contact with outside air.

Examples of a constituent material of the barrel 20 include various resins such as a polyolefin including polyethylene and polypropylene, polystyrene, polyamide, polycarbonate, polyvinyl chloride, poly-(4-methylpentene-1), an acrylic resin, an acrylonitrile-butadiene-styrene copolymer, a polyester including polyethylene terephthalate, a cyclic polyolefin polymer, and a cyclic olefin copolymer. Among these resins, resins such as polypropylene, a cyclic polyolefin polymer, and a cyclic olefin copolymer are preferable because the resins are easily molded and have heat resistance. Note that a cyclic olefin polymer or a cyclic olefin copolymer having high transparency such that a drug solution filled inside can be visually confirmed from the outside and having heat resistance capable of withstanding high-pressure steam sterilization is particularly preferable as a material for forming the barrel 20.

The seal cap 13 includes a body portion 18 having a proximal end side hollow portion that houses the nozzle portion 23 of the barrel 20, and a seal member 19 housed in the body portion 18 and disposed at a distal end portion of the proximal end side hollow portion. In addition, on an outer surface of the proximal end side hollow portion, a cap side screwing portion (male screwing portion) capable of being screwed with the barrel side screwing portion (female screwing portion) formed on an inner surface of the collar 25 is formed.

Examples of a material for forming the seal cap include various resins such as polypropylene, polyethylene, polystyrene, polyamide, polycarbonate, polyvinyl chloride, poly-(4-methylpentene-1), an acrylic resin, an acrylonitrile-butadiene-styrene copolymer, a polyester including polyethylene terephthalate, and a cyclic polyolefin. Among these resins, resins such as polypropylene and a cyclic polyolefin are preferable because the resins are easily molded and have heat resistance.

As a material for forming the seal member 19, an elastic material such as a natural rubber, a synthetic rubber including an isoprene rubber, a butyl rubber, a butadiene rubber, a fluororubber, and a silicone rubber, or a thermoplastic elastomer including an olefin-based elastomer and a styrene-based elastomer is preferable.

The gasket 11 housed in the barrel 20 is made of an elastic rubber or a synthetic resin. The gasket 11 includes a substantially cylindrical body portion extending with substantially the same outer diameter and a plurality of annular ribs disposed on an outer surface of the body portion. Outer surfaces of the annular ribs are in liquid-tight contact with an inner surface of the barrel 20 and are slidable in a liquid-tight state.

As a material for forming the gasket 11, it is preferable to use an elastic rubber (for example, a butyl rubber, a latex rubber, or a silicone rubber), a synthetic resin (for example, a styrene-based elastomer such as an SBS elastomer or an SEBS elastomer, or an olefin-based elastomer such as an ethylene-α olefin copolymer elastomer), or the like.

The plunger 12 is made of a hard or semi-hard resin. In this embodiment, the plunger 12 includes a small disk-shaped gasket pressing portion 14 disposed at a distal end thereof, a disk-shaped pressing operation portion 15 disposed at a proximal end thereof, and a shaft portion 16 having a cross-shaped cross section and extending in the axial direction of the barrel 20 between the gasket pressing portion 14 and the pressing operation portion 15. Note that the shaft portion 16 may be a columnar shaft. The columnar shaft may have a rod shape, a circular columnar shape, a polygonal columnar shape, a circular cylindrical shape, a polygonal cylindrical shape, or the like.

In this embodiment, the plunger 12 is not connected to the gasket 11, and can abut on a proximal end portion of the gasket 11 at the gasket pressing portion 14. The gasket 11 can be moved to a distal end side by pressing after abutment.

A distal end side portion (the gasket pressing portion 14 and a part of a distal end side of the shaft portion 16) of the plunger 12 is housed in the barrel 20 (cylindrical body 21). The plunger 12 includes a stopper portion 17 that is disposed in a portion (shaft portion 16) housed in the barrel 20, abuts on the grip 30 (stopper abutment portion 31) described in detail later, and restricts detachment of the plunger 12 from the barrel 20. More specifically, the stopper portion 17 has a disk shape having an outer shape (outer diameter) smaller than the inner shape (inner diameter) of the barrel 20 (cylindrical body 21) and larger than the outer shape (maximum diameter portion) of the shaft portion 16 of the plunger 12.

As a constituent material of the plunger 12, a hard or semi-hard resin such as high-density polyethylene, polypropylene, polystyrene, or polyethylene terephthalate is preferably used.

In the syringe 1, the drug (drug solution) 60 is stored (filled) in the barrel 20 (in a space formed between the seal cap 13 and the gasket 11).

The drug 60 to be stored is not particularly limited, and examples thereof include a vitamin, a sugar, an electrolyte, an organic acid, a mineral, a fat emulsion, an amino acid, a protein, and an organ preparation mainly as nutrients. Examples thereof further include a drug for a central nervous system, such as a general anesthetic, an antipyretic analgesic, or a general cold preparation, a drug for a peripheral nervous system, such as a local anesthetic or a muscle relaxant, a drug for a sensory organ, such as an ophthalmic agent, a metabolic drug such as a circulatory drug, a respiratory drug, a digestive drug, an urogenital anal drug, a hormonal agent, an antibiotic, or a diabetes drug, a biological preparation such as an antitumor drug, an allergic drug, an antibacterial agent, or an antiviral agent, and a prescription drug such as distilled water or physiological saline mainly as therapeutic agents. Examples thereof further include a vaccine mainly as a preventive agent and a contrast agent mainly as a diagnostic agent.

Many of the exemplified drugs each contain, as a main component, a synthetic low molecular weight compound, a synthetic middle molecular weight compound, a biopharmaceutical such as a polypeptide preparation or a protein preparation, or a biologically derived pharmaceutical such as a blood preparation, but these substances can be used as drugs regardless of the applications described above. In addition, a substance corresponding to a molecular target drug such as an antibody can also be used as a drug. A dosage form of each of these drugs is preferably a liquid even in a drug having a solid dosage form such as a powder or a granule in addition to a liquid from a viewpoint of usability.

In addition, as the drug 60, an ophthalmic drug that can be administered by puncture can be used. Examples of a specific target disease for which such an ophthalmic drug is used include choroidal neovascularization, age-related macular degeneration (both exudative and atrophic), macular edema secondary to retinal vein occlusion (RVO) (including both branch retinal vein occlusion (bRVO) and central retinal vein occlusion (cRVO)), choroidal neovascularization secondary to pathologic myopia (PM), diabetic macular edema (DME), diabetic retinopathy, and proliferative retinopathy. Examples of the drug to be used include ranibizumab [trade name: LUCENTIS (registered trademark)], bevacizumab [trade name: AVASTIN (registered trademark)], and aflibercept [trade name: EYLEA (registered trademark)], which are anti-VEGF antibodies used as therapeutic agents for age-related macular degeneration, and conbercept known as VEGF-TrapEye (aflibercept intravitreal injection solution).

The structure of the syringe barrel grip 30 of the present invention will be described with reference to the embodiment illustrated in Figs. 1 to 14.

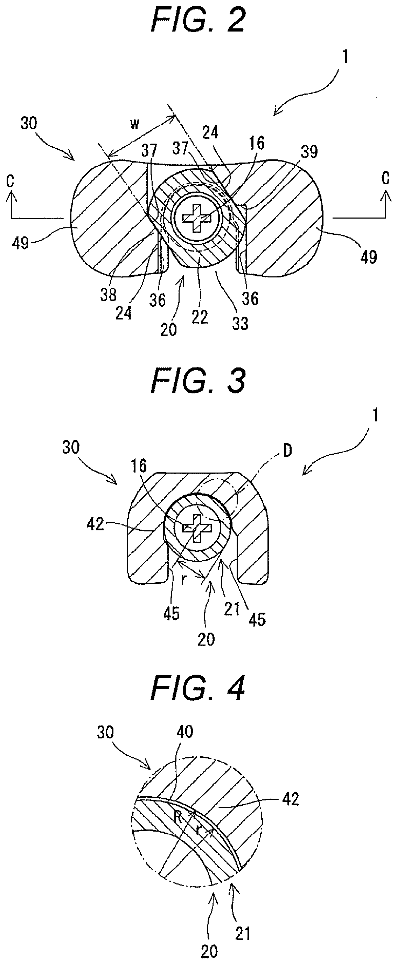

The syringe barrel grip 30 of this embodiment includes a flange housing portion 32 that houses the flange 22. As illustrated in Fig. 2, the flange housing portion 32 includes: a side surface opening 33 formed on a side surface of the grip 30; a flange insertion portion 34 extending in an internal direction of the grip 30 from the side surface opening 33; and a flange rotation restricting portion 35 that communicates with the flange insertion portion 34, extends into the grip 30, and restricts relative rotation between the grip 30 and the flange 22 that has entered the flange rotation restricting portion 35 by rotating the barrel 20 by a predetermined angle in a state where the flange 22 is housed in the flange insertion portion 34. The flange insertion portion 34 includes a first pair of grip straight portions 36 and 36 having a slightly wider width W1 than the pair of flange straight portions 24 and 24 (width w), and the flange rotation restricting portion 35 includes a second pair of grip straight portions 37 and 37 having a slightly wider width W2 than the pair of flange straight portions 24 and 24 (width w) and is inclined with respect to the first pair of grip straight portions 36 and 36.

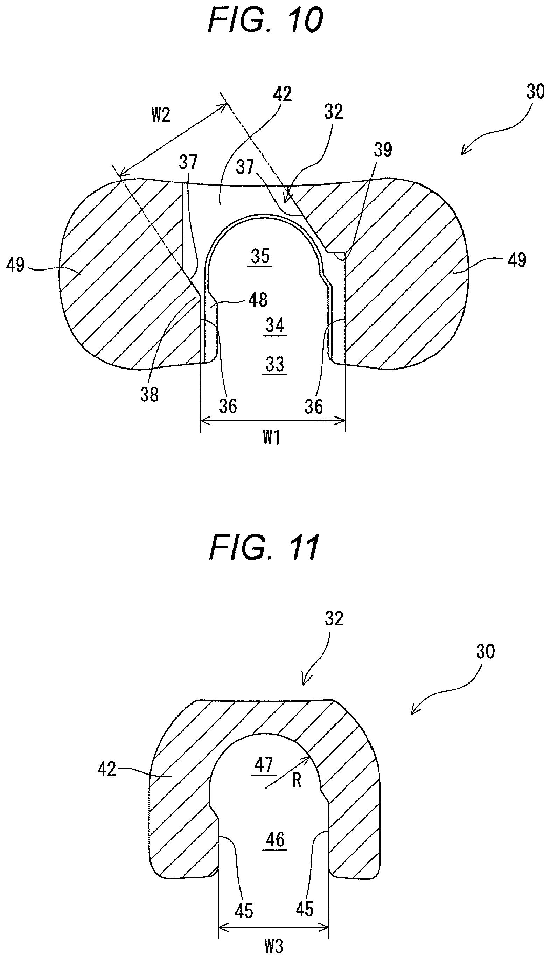

In the present embodiment, as illustrated in Figs. 2, 10, and 12 to 14, at an end portion of the flange insertion portion 34 on the flange rotation restricting portion 35 side, a protrusion 38 that abuts on the flange 22 (flange straight portion 24) when the barrel 20 is rotated and the flange 22 is caused to enter the flange rotation restricting portion 35 is formed. As illustrated in Fig. 10, in the grip 30, at an end portion of the left first grip straight portion 36 (in Fig. 10) of the flange insertion portion 34 on the flange rotation restricting portion 35 side (a connecting portion between the left first grip straight portion 36 and the left second grip straight portion 37), the protrusion 38 is formed. In addition, as illustrated in Fig. 10, between the right first grip straight portion 36 (in Fig. 10) and the right second grip straight portion 37, a stepped portion 39 which is continuous to the first grip straight portion 36 at a right angle and connects the first grip straight portion 36 and the second grip straight portion 37 to each other is formed.

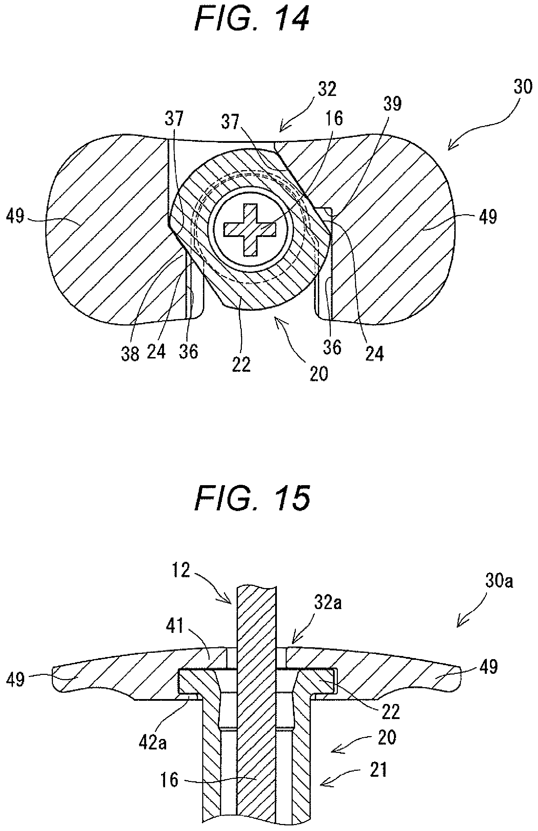

As illustrated in Figs. 2 and 14, in the grip 30, in a state where the flange 22 is housed in the flange rotation restricting portion 35, front sides of the pair of flange straight portions 24 and 24 are located in the second pair of grip straight portions 37 and 37 (between the second pair of grip straight portions 37 and 37), and rear sides of the pair of flange straight portions 24 and 24 are located in the first pair of grip straight portions 36 and 36 (between the first pair of grip straight portions 36 and 36).

Furthermore, in this embodiment, one rear end portion of the pair of flange straight portions 24 and 24 (an end portion of the flange 22 on a rear side in an insertion direction into the grip 30) abuts on or approaches one of the first pair of grip straight portions 36 and 36. That is, as illustrated in Figs. 2 and 14, in Figs. 2 and 14, a rear end portion of the right flange straight portion 24 abuts on or approaches the right first grip straight portion 36. As a result, the flange 22 housed in the flange rotation restricting portion 35 is prevented from moving (rattling) along the second pair of grip straight portions 37 and 37 and being detached from the flange rotation restricting portion 35.

As illustrated in Figs. 3 and 4, in the grip 30, in a state where the flange 22 of the barrel 20 is housed in the flange rotation restricting portion 35 of the grip 30, a lower inner peripheral surface of the grip 30 is not in contact with the barrel 20, and there is a gap 40 for allowing a sterilization gas to flow between the lower inner peripheral surface of the grip 30 and the barrel 20.

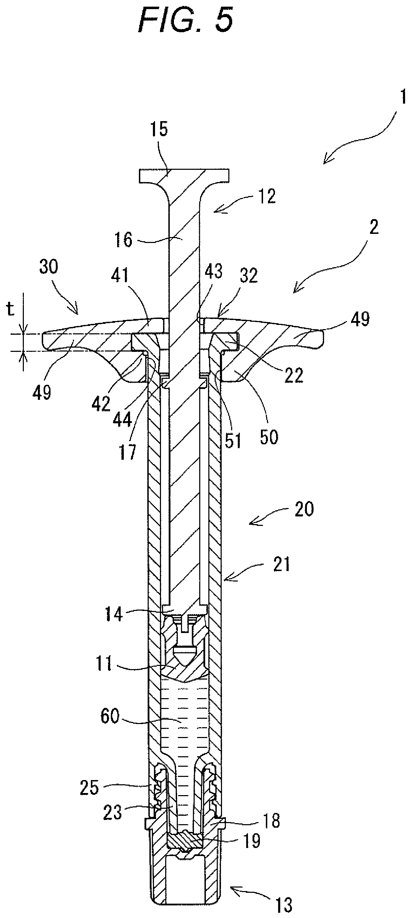

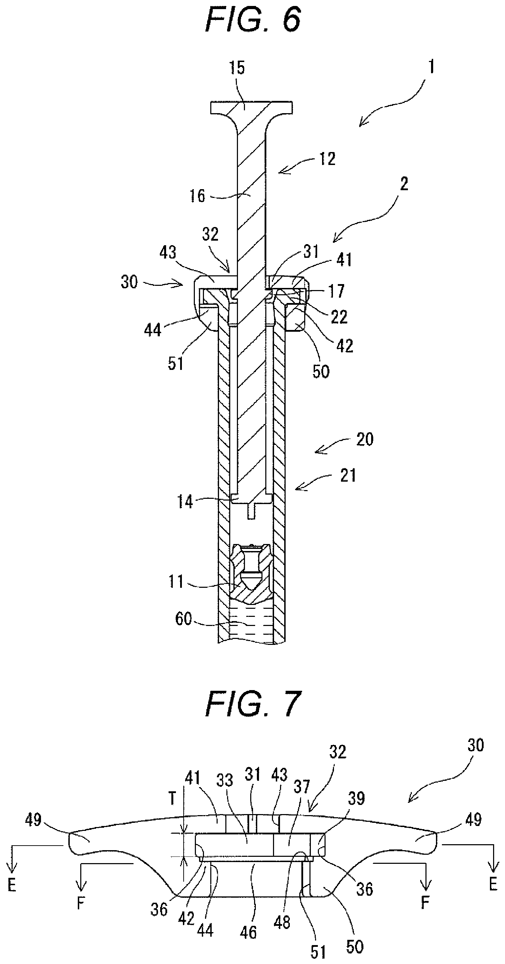

More specifically, as illustrated in Fig. 7, the flange housing portion 32 includes an upper plate portion 41 covering a proximal end side surface of the flange 22 and a lower plate portion 42 covering a distal end side surface of the flange 22. As illustrated in Figs. 5 and 7, a distance T between the upper plate portion 41 and the lower plate portion 42 of the flange housing portion 32 is larger than the thickness t of the flange 22. As a result, a gap for allowing a sterilization gas to flow is formed between the upper plate portion 41 and the proximal end side surface of the flange 22 and/or between the lower plate portion 42 and the distal end side surface of the flange 22.

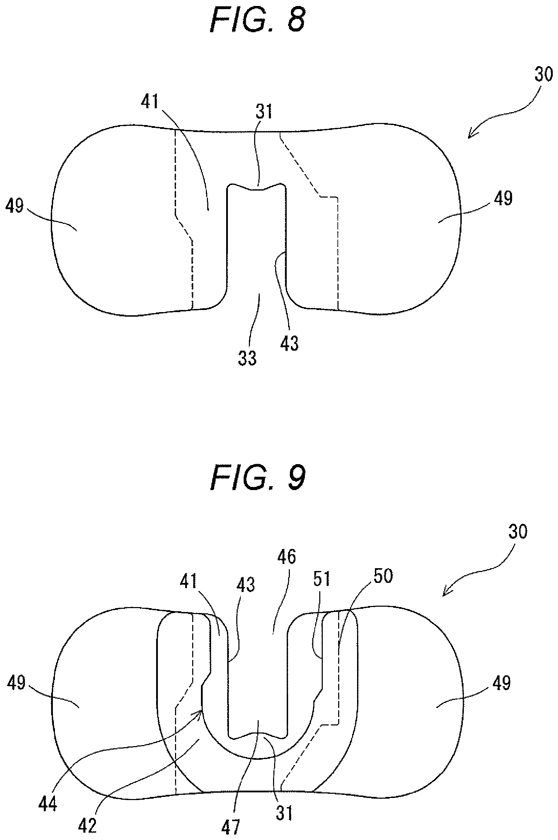

As illustrated in Fig. 8, an upper slit (opening) 43 is formed in the upper plate portion 41 of the flange housing portion 32. The upper slit 43 communicates with the side surface opening 33, the flange insertion portion 34, and the flange rotation restricting portion 35 of the flange housing portion 32, and is formed such that the shaft portion 16 of the plunger 12 can enter the upper slit 43. A part of an inner edge portion of the upper slit 43 is a stopper abutment portion 31 that protrudes inward and abuts on the stopper portion 17 of the plunger 12.

As a result, the upper slit 43 allows the shaft portion 16 of the plunger 12 to pass therethrough, and abuts on the stopper portion 17 of the plunger 12 at the stopper abutment portion 31. Therefore, as illustrated in Fig. 6, the plunger 12 housed in the barrel 20 abuts on the stopper abutment portion 31 of the grip 30 at the stopper portion 17, whereby detachment of the plunger 12 from the barrel 20 is restricted.

As illustrated in Figs. 9 and 10, a lower slit (opening) 44 is formed in the lower plate portion 42 of the flange housing portion 32. The lower slit 44 communicates with the side surface opening 33, the flange insertion portion 34, and the flange rotation restricting portion 35 of the flange housing portion 32, and is formed such that the cylindrical body 21 of barrel 20 can enter the lower slit 44. More specifically, the lower plate portion 42 (lower slit 44) includes a barrel insertion portion 46 including a pair of lower straight portions 45 and 45 that has a width W3 larger (wider) than the outer shape (outer diameter) of the barrel 20 (cylindrical body 21) portion that enters the lower slit 44 and narrower than the width w of the pair of flange straight portions 24 and 24, and are substantially parallel to the first pair of grip straight portions 36 and 36, respectively.

As illustrated in Fig. 11, the lower plate portion 42 (lower slit 44) includes a barrel housing portion 47 that communicates with the barrel insertion portion 46, and partially surrounds the barrel 20 (cylindrical body 21) (houses the barrel 20) in a state where the flange 22 is housed in the flange rotation restricting portion 35. The barrel housing portion 47 has an inner shape (inner diameter, radius R illustrated in Fig. 11) larger than the outer shape (outer diameter, radius r illustrated in Fig. 3) of the barrel 20 (cylindrical body 21) housed therein. As a result, as illustrated in Fig. 3, in a state where the flange 22 is housed in the flange rotation restricting portion 35 of the grip 30, an inner peripheral surface of the barrel housing portion 47 is not in contact with the barrel 20, and the gap 40 for allowing a sterilization gas to flow between the inner peripheral surface of the barrel housing portion 47 and the barrel 20 is formed. That is, in the grip 30, on an inner peripheral surface of the lower plate portion 42 of the flange housing portion 32 (the barrel housing portion 47 of the lower slit 44), at least a part of a lower inner peripheral surface of the grip 30 is formed.

Note that, as illustrated in Figs. 7 and 10, a recess 48 is formed in an upper inner edge portion of the lower plate portion 42. As a result, a sterilization gas can be brought into contact with a wider area of a distal end side surface of the flange 22 facing the lower plate portion 42.

In the flange housing portion 32 of the grip 30, a pair of finger hook portions 49 and 49 is formed so as to constitute a proximal end side portion of the grip 30 and to protrude outward (in a direction orthogonal to the axial direction of the barrel 20). The pair of finger hook portions 49 and 49 protrude in directions opposite to each other and orthogonal to the axial direction of the barrel 20. The pair of finger hook portions 49 and 49 extends outward from the flange 22 of the barrel 20. The pair of finger hook portions 49 and 49 can be used by an operator to hook a finger when the operator operates the syringe 1.

As illustrated in Figs. 5 and 7, the grip 30 of the present embodiment includes a side wall portion 50 extending to an axial distal end side from the flange housing portion 32. The side wall portion 50 is integrated with (connected to) an inner edge portion of the lower slit 44 of the lower plate portion 42 of the flange housing portion 32 at a proximal end thereof. As illustrated in Fig. 7, the outer shape of the side wall portion 50 increases toward a proximal end side (flange housing portion 32 side), and is smoothly connected to the finger hook portions 49 and 49 of the flange housing portion 32. As a result, the side wall portion 50 contributes to improvement in operability of the syringe 1.

An inner peripheral surface of the side wall portion 50 is formed by extending an inner peripheral surface of the lower slit 44 in the axial direction over the entire length in the axial direction, and has a semi-cylindrical shape with a substantially U-shaped cross section as a whole. In the side wall portion 50, an opening for insertion extending over the entire length in the axial direction (for insertion of a proximal end portion of the cylindrical body 21) (insertion slit) 51 is formed. In a state where the flange 22 of the barrel 20 is housed in the flange rotation restricting portion 35 of the grip 30, an inner peripheral surface of the side wall portion 50 is not in contact with the barrel 20, and a gap for allowing a sterilization gas to flow between the inner peripheral surface of the side wall portion 50 and the barrel 20 (the gap 40 and a gap continuous with the gap 40) is formed. In other words, the side wall portion 50 partially covers a proximal end portion of the cylindrical body 21 adjacent to the flange 22, and at least a part of the inner peripheral surface constitutes a lower inner peripheral surface of the grip 30 that forms a gap for allowing a sterilization gas to flow between the inner peripheral surface and the barrel 20.

Preferable examples of a constituent material of the grip 30 include a hard or semi-hard resin such as a polyolefin including polyethylene and polypropylene, polystyrene, polyamide, polycarbonate, polyvinyl chloride, poly-(4-methylpentene-1), an acrylic resin, an acrylonitrile-butadiene-styrene copolymer, a polyester including polyethylene terephthalate, a cyclic polyolefin polymer, or a cyclic olefin copolymer.

Note that, as a constituent material of the grip 30, a material having a thermal expansion coefficient (linear thermal expansion coefficient) larger than that of the barrel 20 may be used. As a result, when the syringe 1 is heated in a surface sterilization (gas sterilization) step described later, an inner surface (inner diameter) of the grip 30 expands more than an outer surface (outer diameter) of the barrel 20 (cylindrical body 21), a gap is formed or expanded between the inner surface of the grip 30 and the outer surface of the cylindrical body 21, and a sterilization gas enters the gap, whereby sterilizability of the syringe 1 can be improved.

A method (process) for manufacturing such a syringe 1 will be described.

First, the barrel 20 is sterilized with the seal cap 13 attached to the nozzle portion 23 of the barrel 20. Note that a sterilization method adopted at this time is not particularly limited, and for example, an autoclave sterilization method using an autoclave (high temperature steam sterilization method), a surface sterilization method using a sterilization gas such as hydrogen peroxide or EOG (gas sterilization method), or a radiation sterilization method by radiation irradiation using a γ ray, an electron radiation, or the like can be used. Here, as an example, autoclave sterilization is performed.

Subsequently, under a sterile environment, the drug 60 that has been previously subjected to sterilization treatment (for example, filtration sterilization) is aseptically filled in the sterilized barrel 20. In the present embodiment, the drug 60 for ophthalmic use, which is relatively vulnerable to heat, is filled. After the drug 60 is filled, the gasket 11 is inserted into the barrel 20. The gasket 11 can be inserted (capped) in a depressurized state.

Subsequently, a distal end side portion of the plunger 12 is housed in the barrel 20, and the grip 30 is attached to the barrel 20. Note that this operation can also be performed under an environment other than a sterile environment.

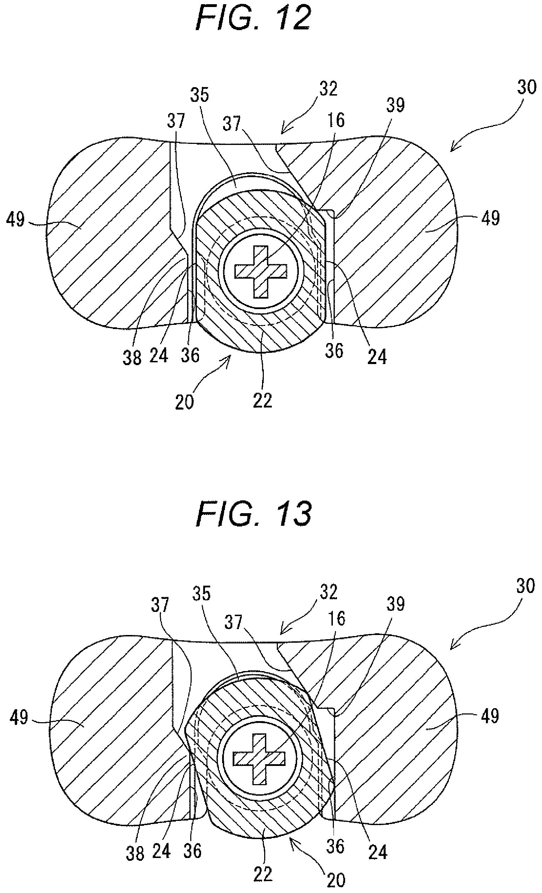

A step of attaching the grip 30 to the barrel 20 will be described in detail with reference to Figs. 12 to 14.

First, as illustrated in Fig. 12, in a state where the pair of flange straight portions 24 and 24 of the barrel 20 and the first pair of grip straight portions 36 and 36 of the grip 30 are substantially parallel to each other, the flange 22 of the barrel 20 is inserted into the grip 30 from the side surface opening 33, and the flange 22 is housed in the flange insertion portion 34.

Subsequently, in a state where the flange 22 is housed in the flange insertion portion 34, the barrel 20 is biased in a rotational direction (here, counterclockwise) and rotated. During the rotation of the barrel 20, as illustrated in Fig. 13, one (here, the left flange straight portion 24 in Fig. 13) of the pair of flange straight portions 24 and 24 abuts on the protrusion 38, and a rear end portion of the other (here, the right flange straight portion 24 in Fig. 13) or a portion in the vicinity thereof abuts on the right first grip straight portion 36 in Fig. 13 [the grip 30 (flange housing portion 32) and barrel 20 (flange 22) are temporarily engaged with each other (first engagement state)].

When the barrel 20 is further biased in the rotational direction from the state where the grip 30 and the barrel 20 are engaged with each other, the upper slit 43, the lower slit 44, and the insertion slit 51 are formed in the grip 30 (the flange housing portion 32 and the side wall portion 50), and therefore the flange housing portion 32 of the grip 30 is elastically deformed and pushed out. As a result, the rotation of the barrel 20 is allowed, the engagement state between the barrel 20 (flange 22) and the grip 30 (flange housing portion 32) is released, and the flange 22 enters the flange rotation restricting portion 35. That is, the grip 30 is temporarily engaged with the barrel 20 when the flange 22 is caused to enter the flange rotation restricting portion 35 by rotating the barrel 20 by a predetermined angle in a state where the flange 22 is housed in the flange insertion portion 34.

As illustrated in Fig. 14, when the flange 22 is housed in the flange rotation restricting portion 35 (the grip 30 is attached to the barrel 20), the grip 30 returns to the original state by an elastic restoring force. By causing the flange 22 to enter the flange rotation restricting portion 35, the attachment of the grip 30 to the barrel 20 is completed. In a state where the flange 22 is housed in the flange rotation restricting portion 35, the pair of flange straight portions 24 and 24 face the second pair of grip straight portions 37 and 37, respectively, whereby relative rotation between the barrel 20 and the grip 30 is restricted.

Note that, although not illustrated, when the barrel 20 is biased in a rotational direction (here, clockwise) opposite to the rotational direction when the flange 22 is caused to enter the flange rotation restricting portion 35 in a state where the grip 30 is attached, one (here, the left flange straight portion 24 in Fig. 14) of the pair of flange straight portions 24 and 24 abuts on the protrusion 38, and a front end portion of the other (here, the right flange straight portion 24 in Fig. 14) or a portion in the vicinity thereof abuts on the right second grip straight portion 37 in Fig. 14 [the grip 30 (flange housing portion 32) and barrel 20 (flange 22) are temporarily engaged with each other (second engagement state)]. Therefore, it is possible to prevent the grip 30 attached to the barrel 20 from unintentionally falling off. In other words, since the grip 30 is temporarily engaged with the barrel 20 (the first engagement state exists) when the flange 22 is caused to enter the flange rotation restricting portion 35 by rotating the barrel 20 by a predetermined angle in a state where the flange 22 is housed in the flange insertion portion 34, the second engagement state occurs in the flange 22 once housed in the flange pivoting restriction portion 35, and detachment of the grip 30 from the barrel 20 after attachment can be prevented.

Subsequently, the syringe 1 with the grip 30 attached to the barrel 20 is sterilized. In the present embodiment, since the drug 60 stored in the barrel 20 is a drug for ophthalmic use, which is relatively vulnerable to heat, surface sterilization (NO₂ sterilization) using a sterilization gas (here, NO₂ (nitrogen dioxide)) is performed.

As described above, the syringe 1 can be aseptically manufactured.

The grip 30 (flange housing portion 32) of the present embodiment includes the flange rotation restricting portion 35 including the second pair of grip straight portions 37 and 37 that is inclined with respect to the first pair of grip straight portions 36 and 36 disposed in the flange insertion portion 34, and in a state where the flange 22 is stored in the flange rotation restricting portion 35, a part of the pair of flange straight portions 24 and 24 is located in the second pair of grip straight portions 37 and 37. Therefore, relative rotation between the flange 22 (barrel 20) and the grip 30 is restricted in a state where the grip 30 is attached to the syringe barrel 20, and therefore operability is good.

Furthermore, in the grip 30 of the present embodiment, in a state where the flange 22 of the barrel 20 is housed in the flange rotation restricting portion 35 of the grip 30, a lower inner peripheral surface of the grip 30 (an inner peripheral surface of the lower slit 44 of the lower plate portion 42 and/or an inner peripheral surface of the side wall portion 50) is not in contact with the barrel 20, and there is a gap 40 for allowing a sterilization gas to flow between the lower inner peripheral surface of the grip 30 and the barrel 20. Therefore, the sterilization gas can be brought into contact with a wider area of surfaces of the grip 30 and the barrel 20, and the syringe barrel grip 30 and the syringe barrel 20 to which such a grip 30 is attached are more reliably sterilized.

Note that, in the present embodiment, the cylindrical body 21 of the barrel 20 has a circular cylindrical shape in which the cross sections of the outer shape and the inner shape are substantially circular, but the present invention is not limited thereto. It is possible to appropriately use those having various cylindrical shapes in which the cross sections are elliptical, polygonal, and the like, those having different cross-sectional shapes between the outer shape and the inner shape, and the like, and the shape of the grip 30 (flange housing portion 32) can also be appropriately changed accordingly.

In addition, as in a grip 30a illustrated in Fig. 15, the side wall portion (50) does not have to be disposed. In the grip 30a, a lower plate portion 42a of a flange housing portion 32a constitutes a most distal end portion (lowest portion) of the grip 30a. As a result, the grip 30a can be further reduced in size and weight.

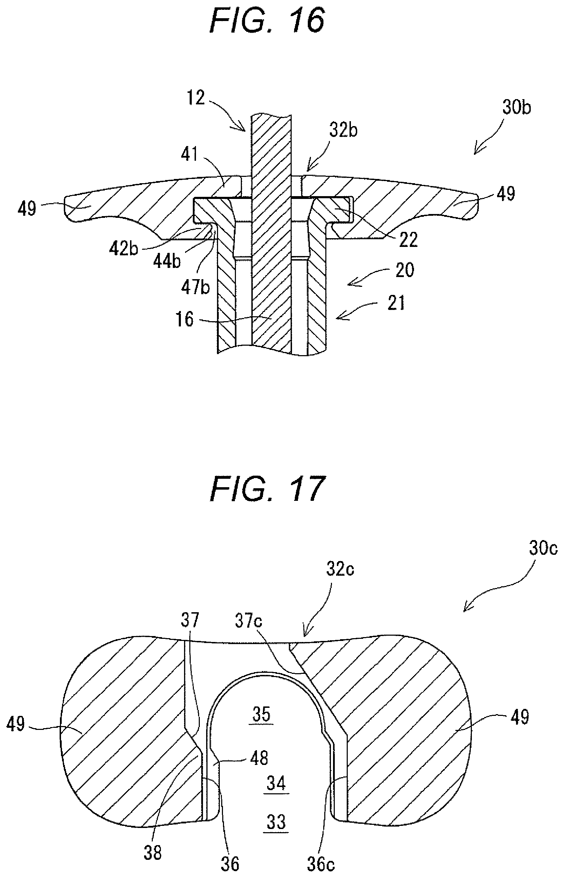

In a grip 30b illustrated in Fig. 16, a lower plate portion 42b of a flange housing portion 32b is thicker than the grip 30a (lower plate portion 42a) described above. As a result, the strength of the grip 30b can be increased. In the grip 30b, as illustrated in Fig. 16, an inner peripheral surface (inner shape) of the lower plate portion 42b (a barrel housing portion 47b of a lower slit 44b) expands downward. As a result, a flow of a sterilization gas between the grip 30b and the barrel 20 is further promoted while the strength of the grip 30b is increased by thickening the lower plate portion 42b, and the sterilization is more reliably performed.

In addition, as in a grip 30c illustrated in Fig. 17, a right first grip straight portion 36c and a right second grip straight portion 37c in Fig. 17 in a flange housing portion 32c may be connected to each other as they are without forming the stepped portion (39).

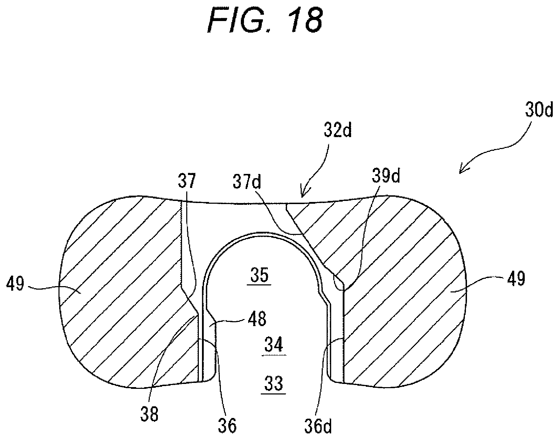

In addition, as in a grip 30d illustrated in Fig. 18, a right first grip straight portion 36d and a right second grip straight portion 37d in Fig. 18 in a flange housing portion 32d may be connected to each other at a gentle angle with an inclined stepped portion 39d.

In addition, although not illustrated, the protrusion 38 formed in the flange housing portion 32 is not limited to the exemplified one. For example, the protrusion 38 may be formed so as to protrude from the first grip straight portion 36 toward the inside of the flange housing portion 32, and as for a formation portion thereof, the protrusion 38 may be formed at a middle portion of the first grip straight portion 36 instead of the end portion of the first grip straight portion 36 on the flange rotation restricting portion 35 side.

### Industrial Applicability

The syringe barrel grip of the present invention is attached to a syringe barrel including a cylindrical body and a flange disposed in the cylindrical body, protruding outward, and including a pair of flange straight portions facing each other in parallel. The grip of the present invention includes a flange housing portion that houses a flange, and the flange housing portion includes: a side surface opening formed on a side surface of the grip; a flange insertion portion extending in an internal direction of the grip from the side surface opening; and a flange rotation restricting portion that communicates with the flange insertion portion, extends into the grip, and restricts relative rotation between the grip and the flange that has entered the flange rotation restricting portion by rotating the barrel by a predetermined angle in a state where the flange is housed in the flange insertion portion.

Furthermore, the flange insertion portion of the grip of the present invention includes a first pair of grip straight portions having a slightly wider width than the pair of flange straight portions, the flange rotation restricting portion includes a second pair of grip straight portions having a slightly wider width than the pair of flange straight portions and is inclined with respect to the first pair of grip straight portions, and in a state where the flange is housed in the flange rotation restricting portion, front sides of the pair of flange straight portions are located in the second pair of grip straight portions, and rear sides of the pair of flange straight portions are located in the first pair of grip straight portions.

Therefore, in the syringe barrel grip, the barrel assembly, and the syringe of the present invention, relative rotation between the flange and the grip is restricted in a state where the grip is attached to the syringe barrel, and therefore operability is good.

Furthermore, in the grip of the present invention, in a state where the flange of the barrel is housed in the flange rotation restricting portion of the grip, a lower inner peripheral surface of the grip is not in contact with the barrel, and there is a gap for allowing a sterilization gas to flow between the lower inner peripheral surface of the grip and the barrel.

Therefore, in the syringe barrel grip, the barrel assembly, and the syringe of the present invention, the sterilization gas can be brought into contact with a wider area of surfaces of the grip and the barrel, and the syringe barrel grip and a syringe barrel to which such a grip is attached are more reliably sterilized.

Furthermore, the flange insertion portion of the grip includes a first pair of grip straight portions having a slightly wider width than the pair of flange straight portions, the flange rotation restricting portion includes a second pair of grip straight portions having a slightly wider width than the pair of flange straight portions and is inclined with respect to the first pair of grip straight portions, and in a state where the flange is housed in the flange rotation restricting portion, front sides of the pair of flange straight portions are located in the second pair of grip straight portions, and rear sides of the pair of flange straight portions are located in the first pair of grip straight portions.

Therefore, in the barrel assembly of the present invention, relative rotation between the flange and the grip is restricted in a state where the grip is attached to the syringe barrel, and therefore operability is good.

Furthermore, in the barrel assembly of the present invention, in a state where the flange of the barrel is housed in the flange rotation restricting portion of the grip, a lower inner peripheral surface of the grip is not in contact with the barrel, and there is a gap for allowing a sterilization gas to flow between the lower inner peripheral surface of the grip and the barrel.

Therefore, in the barrel assembly of the present invention, the sterilization gas can be brought into contact with a wider area of surfaces of the grip and the barrel, and the syringe barrel grip and a syringe barrel to which such a grip is attached are more reliably sterilized.

The syringe of the present invention includes a syringe barrel grip attached to a syringe barrel including a cylindrical body and a flange disposed in the cylindrical body, protruding outward, and including a pair of flange straight portions facing each other in parallel. The grip includes a flange housing portion that houses a flange, and the flange housing portion includes: a side surface opening formed on a side surface of the grip; a flange insertion portion extending in an internal direction of the grip from the side surface opening; and a flange rotation restricting portion that communicates with the flange insertion portion, extends into the grip, and restricts relative rotation between the grip and the flange that has entered the flange rotation restricting portion by rotating the barrel by a predetermined angle in a state where the flange is housed in the flange insertion portion.

Furthermore, the flange insertion portion of the grip includes a first pair of grip straight portions having a slightly wider width than the pair of flange straight portions, the flange rotation restricting portion includes a second pair of grip straight portions having a slightly wider width than the pair of flange straight portions and is inclined with respect to the first pair of grip straight portions, and in a state where the flange is housed in the flange rotation restricting portion, front sides of the pair of flange straight portions are located in the second pair of grip straight portions, and rear sides of the pair of flange straight portions are located in the first pair of grip straight portions.

Therefore, in the syringe of the present invention, relative rotation between the flange and the grip is restricted in a state where the grip is attached to the syringe barrel, and therefore operability is good.

Furthermore, in the syringe of the present invention, in a state where the flange of the barrel is housed in the flange rotation restricting portion of the grip, a lower inner peripheral surface of the grip is not in contact with the barrel, and there is a gap for allowing a sterilization gas to flow between the lower inner peripheral surface of the grip and the barrel.

Therefore, in the syringe of the present invention, the sterilization gas can be brought into contact with a wider area of surfaces of the grip and the barrel, and the syringe barrel grip and a syringe barrel to which such a grip is attached are more reliably sterilized.

## Claims

1. A syringe barrel grip (30) attached to a syringe barrel (20) including a cylindrical body and (21) a flange (22) disposed in the cylindrical body (21), protruding outward, and including a pair of flange straight portions (24; 24) facing each other in parallel,
the grip (30; 30a; 30b; 30c; 30d) comprising a flange housing portion (32; 32a; 32b; 32c; 32d) that houses the flange (22), wherein
the flange housing portion (32; 32a; 32b; 32c; 32d) includes: a side surface opening (33) formed on a side surface of the grip (30); a flange insertion portion (34) extending in an internal direction of the grip (30) from the side surface opening (33); and a flange rotation restricting portion (35) that communicates with the flange insertion portion (34), extends into the grip (30), and restricts relative rotation between the grip (30) and the flange (22) that has entered the flange rotation restricting portion (35) by rotating the barrel (20) by a predetermined angle in a state where the flange (22) is housed in the flange insertion portion (34),
the flange insertion portion (34) includes a first pair of grip straight portions (36; 36) having a wider width than the pair of flange straight portions (24; 24), and the flange rotation restricting portion (35) includes a second pair of grip straight portions (37; 37) having a wider width than the pair of flange straight portions (24; 24) and is inclined with respect to the first pair of grip straight portions (36; 36),
in a state where the flange (22) is housed in the flange rotation restricting portion (35), front sides of the pair of flange straight portions (24; 24) are located in the second pair of grip straight portions (37; 37), and rear sides of the pair of flange straight portions (24; 24) are located in the first pair of grip straight portions (36; 36), and
in a state where the flange (22) of the barrel (20) is housed in the flange rotation restricting portion (35) of the grip (30), a lower inner peripheral surface of the grip (30) is not in contact with the barrel (20), and there is a gap (40) for allowing a sterilization gas to flow between the lower inner peripheral surface of the grip (30) and the barrel (20).

2. The syringe barrel grip (30) according to claim 1, wherein the grip (30) includes a side wall portion (50) partially covering a proximal end portion of the cylindrical body (21) adjacent to the flange (22), and at least a part of an inner peripheral surface of the side wall portion (50) is the lower inner peripheral surface.

3. The syringe barrel grip (30) according to claim 1 or 2, wherein the grip (30) is temporarily engaged with the barrel (20) when the flange (22) is caused to enter the flange rotation restricting portion (35) by rotating the barrel (20) by a predetermined angle in a state where the flange (22) is housed in the flange insertion portion (34).

4. The syringe barrel grip (30) according to any one of claims 1 to 3, wherein the flange housing portion (32; 32a; 32b; 32c; 32d) includes an upper plate portion (41) covering a proximal end side surface of the flange (22) and a lower plate portion covering a distal end side surface of the flange (22), a distance between the upper plate portion (41) and the lower plate portion is larger than a thickness of the flange (22), and a gap (40) for allowing a sterilization gas to flow is formed between the upper plate portion (41) and the proximal end side surface of the flange (22) and/or between the lower plate portion and the distal end side surface of the flange (22).

5. A barrel assembly (2) comprising: a syringe barrel (20) including a cylindrical body (21) and a flange (22) disposed at a proximal end of the cylindrical body, protruding outward, and including a pair of flange straight portions (24; 24) facing each other in parallel; and the syringe barrel grip (30) according to any one of claims 1 to 4 attached to the barrel (20).

6. A syringe (1) comprising: the barrel assembly (2) according to claim 5; a gasket (11) slidably housed in the barrel (20); and a plunger (12) for moving the gasket (11).

7. The syringe (1) according to claim 6, wherein a distal end side portion of the plunger (12) is housed in the barrel (20), and the plunger (12) includes a stopper portion (17) that is disposed in a portion housed in the barrel (20), abuts on the grip (30), and restricts detachment of the plunger (12) from the barrel (20).

8. The syringe (1) according to claim 6 or 7, wherein the plunger (12) is not connected to the gasket (11) and includes a gasket pressing portion (14) that abuts on a proximal end portion of the gasket (11) at a distal end thereof.

9. The syringe (1) according to any one of claims 6 to 8, wherein a drug (60) is stored in the barrel (20).

## Patentansprüche

1. Spritzenzylindergriff (30), der an einem Spritzenzylinder (20) angebracht ist, der einen zylindrischen Körper (21) und einen Flansch (22) umfasst, der in dem zylindrischen Körper (21) angeordnet ist, nach außen vorsteht und ein Paar gerade Flanschabschnitte (24; 24), welche einander parallel gegenüberliegen, umfasst,
der Griff (30; 30a; 30b; 30c; 30d) einen Flanschgehäuseabschnitt (32; 32a; 32b; 32c; 32d) umfasst, in dem der Flansch (22) untergebracht ist, wobei
der Flanschgehäuseabschnitt (32; 32a; 32b; 32c; 32d) umfasst: eine Seitenflächenöffnung (33), die an einer Seitenfläche des Griffs (30) ausgebildet ist; einen Flanscheinführungsabschnitt (34), der sich von der Seitenflächenöffnung (33) in eine Innenrichtung des Griffs (30) erstreckt; und einen Abschnitt (35) zur Begrenzung der Flanschdrehung, der mit dem Flanscheinführungsabschnitt (34) in Verbindung steht, sich in den Griff (30) erstreckt und die relative Drehung zwischen dem Griff (30) und dem Flansch (22), der in den Abschnitt (35) zur Begrenzung der Flanschdrehung eingetreten ist, durch Drehen des Zylinders (20) um einen vorbestimmten Winkel in einem Zustand begrenzt, in dem der Flansch (22) in dem Flanscheinführungsabschnitt (34) untergebracht ist,
der Flanscheinführungsabschnitt (34) ein erstes Paar von geraden Griffabschnitten (36; 36) umfasst, die eine größere Breite als das Paar von geraden Flanschabschnitten (24; 24) aufweist, und der Abschnitt (35) zur Begrenzung der Flanschdrehung ein zweites Paar von geraden Griffabschnitten (37; 37) umfasst, die eine größere Breite als das Paar von geraden Flanschabschnitten (24; 24) aufweist, und in Bezug auf das erste Paar von geraden Griffabschnitten (36; 36) geneigt ist,
in einem Zustand, in dem der Flansch (22) in dem Abschnitt (35) zur Begrenzung der Flanschdrehung untergebracht ist, sind die Vorderseiten des Paares von geraden Flanschabschnitten (24; 24) in dem zweiten Paar von geraden Griffabschnitten (37; 37) angeordnet und die Rückseiten des Paares von geraden Flanschabschnitten (24; 24) sind in dem ersten Paar von geraden Griffabschnitten (36; 36) angeordnet, und
in einem Zustand, in dem der Flansch (22) des Zylinders (20) in dem Abschnitt (35) zur Begrenzung der Flanschdrehung des Griffs (30) untergebracht ist, steht eine untere innere Umfangsfläche des Griffs (30) nicht mit dem Zylinder (20) in Kontakt und es ist ein Spalt (40) vorhanden, um es einem Sterilisationsgas zu ermöglichen, zwischen der unteren inneren Umfangsfläche des Griffs (30) und dem Zylinder (20) zu strömen.

2. Spritzenzylindergriff (30) nach Anspruch 1, wobei der Griff (30) einen Seitenwandabschnitt (50) umfasst, der teilweise einen proximalen Endabschnitt des zylindrischen Körpers (21) benachbart zu dem Flansch (22) bedeckt, und mindestens ein Teil einer inneren Umfangsfläche des Seitenwandabschnitts (50) die untere innere Umfangsfläche ist.

3. Spritzenzylindergriff (30) nach Anspruch 1 oder 2, wobei der Griff (30) vorübergehend mit dem Zylinder (20) in Eingriff steht, wenn der Flansch (22) durch Drehen des Zylinders (20) um einen vorbestimmten Winkel in einem Zustand, in dem der Flansch (22) in dem Flanscheinführungsabschnitt (34) untergebracht ist, veranlasst wird, in den Abschnitt (35) zur Begrenzung der Flanschdrehung einzutreten.

4. Spritzenzylindergriff (30) nach einem der Ansprüche 1 bis 3, wobei der Flanschgehäuseabschnitt (32; 32a; 32b; 32c; 32d) einen oberen Plattenabschnitt (41), der eine proximale Endseitenfläche des Flansches (22) bedeckt, und einen unteren Plattenabschnitt umfasst, der eine distale Endseitenfläche des Flansches (22) bedeckt, wobei ein Abstand zwischen dem oberen Plattenabschnitt (41) und dem unteren Plattenabschnitt größer ist als eine Dicke des Flansches (22), und zwischen dem oberen Plattenteil (41) und der proximalen Endseitenfläche des Flansches (22) und/oder zwischen dem unteren Plattenteil und der distalen Endseitenfläche des Flansches (22) ist ein Spalt (40) ausgebildet, um es einem Sterilisationsgas zu ermöglichen, dort zu strömen.

5. Zylinderanordnung (2), umfassend: einen Spritzenzylinder (20), der einen zylindrischen Körper (21) und einen Flansch (22) umfasst, der an einem proximalen Ende des zylindrischen Körpers angeordnet ist, nach außen vorsteht und ein Paar gerade Flanschabschnitte (24; 24) umfasst, welche einander parallel gegenüberliegen; und den Spritzenzylindergriff (30) nach einem der Ansprüche 1 bis 4, der an dem Zylinder (20) angebracht ist.

6. Spritze (1), umfassend: die Zylinderanordnung (2) nach Anspruch 5; eine Dichtung (11), die verschiebbar in dem Zylinder (20) untergebracht ist; und einen Kolben (12) zum Bewegen der Dichtung (11).

7. Spritze (1) nach Anspruch 6, wobei ein distaler Endabschnitt des Kolbens (12) in dem Zylinder (20) untergebracht ist, und der Kolben (12) einen Anschlagabschnitt (17) aufweist, der in einem in dem Zylinder (20) untergebrachten Abschnitt angeordnet ist, an dem Griff (30) anliegt und ein Lösen des Kolbens (12) aus dem Zylinder (20) verhindert.

8. Spritze (1) nach Anspruch 6 oder 7, wobei der Kolben (12) nicht mit der Dichtung (11) verbunden ist und einen Dichtungsandrückabschnitt (14) aufweist, der an einem proximalen Endabschnitt der Dichtung (11) an einem distalen Ende davon anliegt.

9. Spritzenanordnung (1) nach einem der Ansprüche 6 bis 8, wobei ein Arzneimittel (60) in dem Zylinder (20) aufbewahrt ist.

## Revendications

1. Poignée (30) de cylindre de seringue fixée à un cylindre (20) de seringue incluant un corps cylindrique (21) et une bride (22) disposée dans le corps cylindrique (21), faisant saillie vers l'extérieur, et incluant une paire de parties droites (24 ; 24) de bride se faisant face en parallèle,
la poignée (30 ; 30a ; 30b ; 30c ; 30d) comprenant une partie de logement (32 ; 32a ; 32b ; 32c ; 32d) de bride qui loge la bride (22), dans laquelle
la partie de logement (32 ; 32a ; 32b ; 32c ; 32d) de bride inclut : une ouverture (33) de surface latérale formée sur une surface latérale de la poignée (30) ; une partie d'insertion (34) de bride s'étendant dans une direction interne de la poignée (30) à partir de l'ouverture (33) de surface latérale ; et une partie limitant la rotation (35) de bride qui communique avec la partie d'insertion (34) de bride, s'étend dans la poignée (30) et empêche la rotation relative entre la poignée (30) et la bride (22) qui a pénétré dans la partie limitant la rotation (35) de bride en faisant tourner le cylindre (20) d'un angle prédéterminé dans un état où la bride (22) est logée dans la partie d'insertion (34) de bride,
la partie d'insertion (34) de bride inclut une première paire de parties droites (36 ; 36) de poignée présentant une plus grande largeur que la paire de parties droites (24 ; 24) de bride, et la partie limitant la rotation (35) de bride inclut une seconde paire de parties droites (37 ; 37) de poignée présentant une plus grande largeur que la paire de parties droites (24 ; 24) de bride et est inclinée par rapport à la première paire de parties droites (36 ; 36) de poignée,
dans un état où la bride (22) est logée dans la partie limitant la rotation (35) de bride, des côtés avant de la paire de parties droites (24 ; 24) de bride sont situés dans la seconde paire de parties droites (37 ; 37) de poignée, et des côtés arrière de la paire de parties droites (24 ; 24) de bride sont situés dans la première paire de parties droites (36 ; 36) de poignée, et
dans un état où la bride (22) du cylindre (20) est logée dans la partie limitant la rotation (35) de bride de la poignée (30), une surface périphérique interne inférieure de la poignée (30) n'est pas en contact avec le cylindre (20), et un espace (40) est présent pour permettre à un gaz de stérilisation de circuler entre la surface périphérique interne inférieure de la poignée (30) et le cylindre (20).

2. Poignée (30) de cyclindre de seringue selon la revendication 1, dans laquelle la poignée (30) inclut une partie de paroi latérale (50) recouvrant partiellement une partie d'extrémité proximale du corps cylindrique (21) de manière adjacente à la bride (22), et au moins une partie d'une surface périphérique interne de la partie de paroi latérale (50) est la surface périphérique interne inférieure.

3. Poignée (30) de cyclindre de seringue selon la revendication 1 ou 2, dans laquelle la poignée (30) est temporairement en prise avec le cylindre (20) lorsque la bride (22) est amenée à pénétrer dans la partie limitant la rotation (35) de bride en faisant tourner le cylindre (20) d'un angle prédéterminé dans un état où la bride (22) est logée dans la partie d'insertion (34) de bride.

4. Poignée (30) de cylindre de seringue selon l'une quelconque des revendications 1 à 3, dans laquelle la partie de logement (32 ; 32a ; 32b ; 32c ; 32d) de bride inclut une partie de plaque supérieure (41) recouvrant une surface latérale d'extrémité proximale de la bride (22) et une partie de plaque inférieure recouvrant une surface latérale d'extrémité distale de la bride (22), une distance entre la partie de plaque supérieure (41) et la partie de plaque inférieure est supérieure à une épaisseur de la bride (22), et un espace (40) permettant à un gaz de stérilisation de circuler est formé entre la partie de plaque supérieure (41) et la surface latérale d'extrémité proximale de la bride (22) et/ou entre la partie de plaque inférieure et la surface latérale d'extrémité distale de la bride (22).

5. Ensemble cylindre (2) comprenant : un cylindre (20) de seringue incluant un corps cylindrique (21) et une bride (22) disposée à une extrémité proximale du corps cylindrique, faisant saillie vers l'extérieur, et incluant une paire de parties droites (24 ; 24) de bride se faisant face en parallèle ; et la poignée (30) de cylindre de seringue selon l'une quelconque des revendications 1 à 4 fixée au cylindre (20).

6. Seringue (1) comprenant : l'ensemble cylindre (2) selon la revendication 5 ; un joint (11) logé de manière coulissante dans le cylindre (20) ; et un piston (12) pour déplacer le joint (11).

7. Seringue (1) selon la revendication 6, dans laquelle une partie latérale d'extrémité distale du piston (12) est logée dans le cylindre (20), et le piston (12) inclut une partie de butée (17) qui est disposée dans une partie logée dans le cylindre (20), vient buter contre la poignée (30) et empêche le piston (12) de se détacher du cylindre (20).

8. Seringue (1) selon la revendication 6 ou 7, dans laquelle le piston (12) n'est pas connecté au joint (11) et inclut une partie de pression (14) de joint qui vient buter contre une partie d'extrémité proximale du joint (11) à une extrémité distale de celui-ci.

9. Seringue (1) selon l'une quelconque des revendications 6 à 8, dans laquelle un médicament (60) est stocké dans le cylindre (20).
